# FASCICULE DE BREVET EUROPEEN

(11) **EP 1 252 912 B1**
(45) Date de publication et mention de la délivrance du brevet: **05.03.2008**
(21) Numéro de dépôt: 02290729.9
(22) Date de dépôt: 22.03.2002
(51) Int. Cl.: A61N 1/372, G06F 19/00, G06F 17/00

(54) **Procédé de traitement de données mémorisées dans un dispositif médical implantable actif pour l'aide au diagnostic par un practicien**
Verfahren zur Verarbeitung von gespeicherten Daten in einer aktiven, implantierbaren, medizinischen Vorrichtung zur Unterstützung einer Ärztlichen Diagnose
Method for processing data stored in an active implantable medical device for helping the making of a diagnosis by a practitioner

(30) Priorité: 29.03.2001 FR 0104260
(43) Date de publication de la demande: 30.10.2002
(73) Titulaire: ELA MEDICAL, 92541 Montrouge (FR)
(72) Inventeur: Graindorge, Laurence, 92290 Chatenay Malabry (FR)
(74) Mandataire: Dupuis-Latour, Dominique

(56) Documents cités:
- US-A- 5 487 755
- US-A- 5 549 654
- US-A- 5 697 959
- US-A- 5 724 985
- US-A- 6 016 442

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs, cardioverteurs et/ou dispositifs multisite pour le traitement des troubles du rythme cardiaque.

Elle concerne plus précisément le traitement de données stockées en mémoire vive (RAM) du dispositif telles qu'histogrammes, courbes, électrogrammes, etc. Ces données stockées dans un implant seront par la suite dénommées "données d'implant".

Ces données d'implant peuvent être lues au moyen d'un programmateur par des techniques de télémétrie en elles-mêmes bien connues. Le programmateur transmet ses données à un ordinateur qui va les traiter et les représenter sous forme de liste et/ou sous forme graphique sur un écran d'affichage, afin d'aider le praticien à analyser l'historique des données cliniques du patient et du fonctionnement du stimulateur sur une période de plusieurs jours ou plusieurs semaines. L'interprétation de ces données lui permettra de poser son diagnostic.

Le US-A-5 724 985 (Snell et al.) décrit un dispositif pourvu d'une interface permettant d'afficher sur un même écran les données recueillies par l'implant sous forme graphique ou de séries de valeurs numériques, et de naviguer entre plusieurs écrans par sélection d'onglets. Des fenêtres surgissantes ("pop up") permettant de sélectionner divers paramètres d'affichage des données.

On connaît diverses techniques d'aide au diagnostic permettant d'élaborer des données de synthèse à partir des données brutes lues dans l'implant, par exemple le logiciel AIDA (Aide à l'Interprétation et au Diagnostic Automatiques) d'ELA Médical, logiciel dont les fonctionnalités sont décrites dans l'article de Limousin et coll., Value of Automatic Processing and Reliability of Stored Data in an Implanted Pacemaker: Initial Results in 59 Patients, PACE;20[Pt. I]:2893-2898 (1997).

On notera que l'invention ne concerne pas la manière dont sont élaborées ces données de synthèse, qui est connue en elle-même, ni le diagnostic proprement dit, qui est posé par le praticien à l'aide des données de synthèse élaborées par le logiciel.

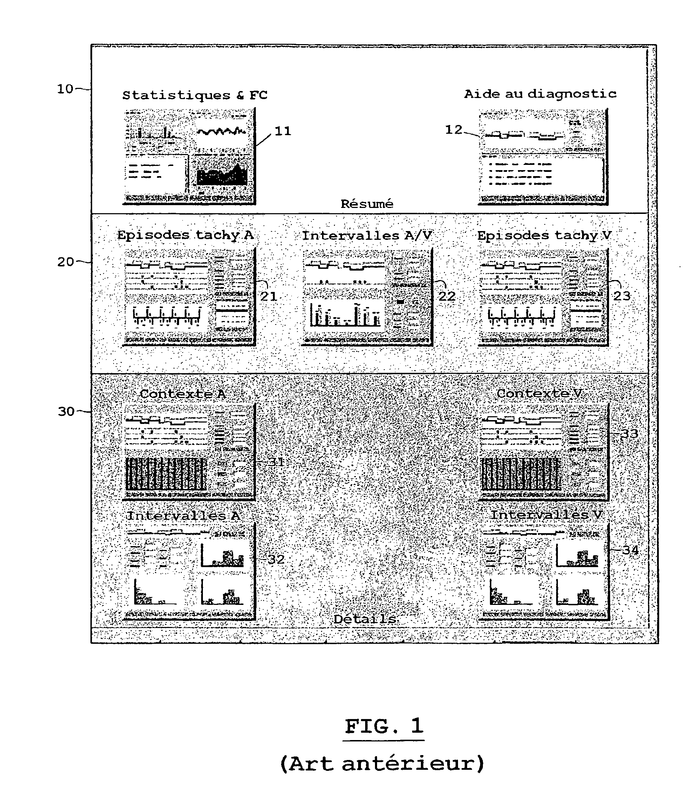

Ces données de synthèse sont, en pratique, présentées au praticien sous forme de séries de pages d'écran, comme illustré par exemple sur la figure 1.

Une première série 10 d'écrans comprend un écran 11 d'affichage de diverses statistiques ainsi que l'évolution dans le temps de la fréquence cardiaque, c'est-à-dire une représentation graphique des données brutes enregistrées dans l'implant. Un écran 12 permet d'afficher un certain nombre de messages et de graphiques d'aide au diagnostic, c'est-à-dire des données de synthèse élaborées par exemple à partir d'un logiciel tel que le logiciel AIDA cité plus haut.

Une deuxième série 20 d'écrans permet d'afficher des informations relatives à des aspects plus particuliers, par exemple un écran 21 pour les épisodes de tachycardie auriculaire, un écran 22 pour les intervalles atrioventriculaires et un écran 23 pour les épisodes de tachycardie ventriculaire.

Enfin, une troisième série 30 d'écrans fournit des informations plus détaillées sur des aspects particuliers comme le contexte auriculaire, les intervalles auriculaires, le contexte ventriculaire ou les intervalles ventriculaires (écrans 31 à 34).

Cette organisation des données affichées est certes très complète, puisqu'elle permet au praticien d'accéder à un très grand nombre d'informations et autoriser ainsi une analyse fine, mais elle présente l'inconvénient d'obliger le praticien à consulter de multiples écrans spécialisés et complexes, nécessitant un très grand nombre de va-et-vients entre écrans différents, tous complexes. Cette incommodité est en outre aggravée pour les praticiens n'ayant pas une pratique très développée du logiciel, notamment ceux qui doivent travailler sur plusieurs logiciels différents (il existe en effet autant de logiciels que de fabricants de stimulateurs), ou bien les cardiologues, moins spécialisés dans l'analyse des données d'implant que les électrophysiologistes, mais qui peuvent avoir intérêt à avoir connaissance de certaines données enregistrées pour les aider dans un diagnostic clinique du malade, pour tenter par exemple de retrouver certains signes révélateurs d'une pathologie particulière.

L'un des buts de l'invention est de proposer un procédé de traitement des données d'implant permettant d'organiser et d'afficher celles-ci d'une manière plus sélective, pour faciliter la consultation par le praticien sans faire pour autant de concessions sur le niveau de détail des données affichables.

Essentiellement, ce procédé consiste à regrouper sur un même écran récapitulatif les données les plus significatives sous forme d'une pluralité de champs, et à permettre au praticien, lorsqu'il choisit et sélectionne un champ donné, de voir apparaître sur ce même écran (par exemple en superposition) des données spécifiques en rapport avec ce champ - et avec ce seul champ.

Plus précisément, le procédé de l'invention est un procédé de traitement de données d'implant caractérisé par les étapes énoncées dans la revendication 1. Les sous-revendications énoncent des mises en oeuvre avantageuses de ce procédé.

L'invention a également pour objet un programmateur apte à coopérer avec un dispositif médical implantable actif conservant dans sa mémoire des données d'implant brutes. Ce programmateur comporte les éléments énoncés dans la revendication 5.

On va maintenant décrire des exemples de mises en oeuvre du procédé de l'invention, en référence aux dessins annexés.

La figure 1, décrite plus haut, illustre les séries d'écrans produits par les procédés de l'art antérieur.

La figure 2 illustre un écran récapitulatif produit par le procédé de l'invention, avant affichage des données spécifiques.

La figure 3 illustre l'écran de la figure 3, avec affichage des données spécifiques suite à la sélection d'un champ d'affichage.

La figure 4 illustre un autre exemple d'écran récapitulatif, avec affichage des données spécifiques relatives à l'un des champs.

La figure 5 est homologue de la figure 4, pour l'affichage des données spécifiques relatives à un autre champ.

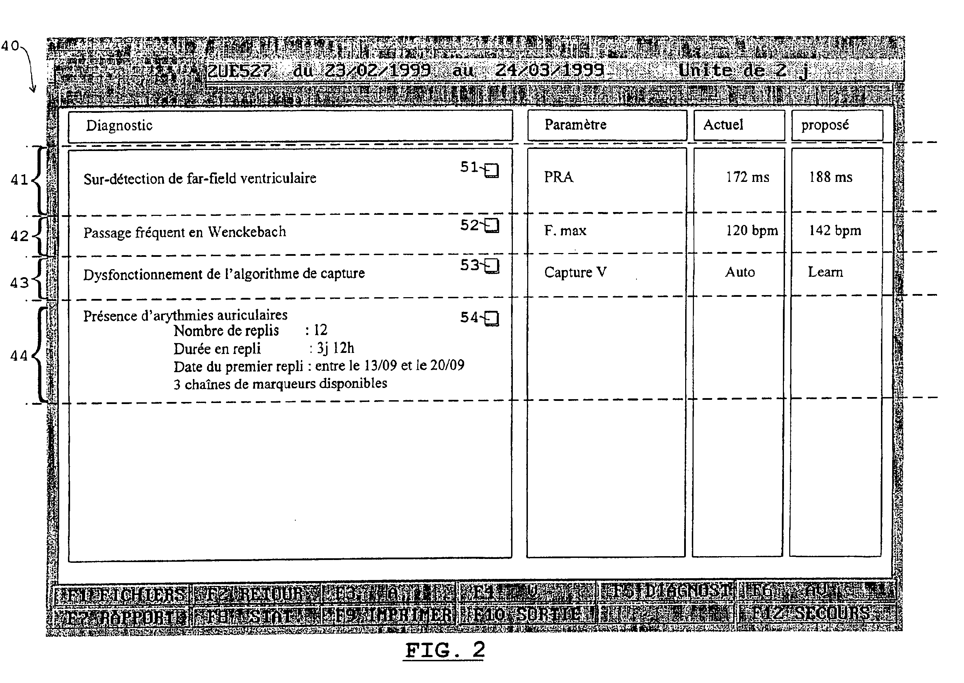

Sur la figure 2, on a illustré un écran récapitulatif 40 produit par le procédé de l'invention, regroupant des données de synthèse élaborées par exemple par mise en oeuvre d'un logiciel tel que le logiciel précité AIDA d'ELA Médical.

Ce logiciel produit un certain nombre de messages d'aide au diagnostic tels que *"Sur-détection de far-field ventriculaire*", *"Passage fréquent en Wenckebach*", *"Dysfonctionnement de l'algorithme de capture",* etc. Plus généralement, les messages d'analyse peuvent comprendre des diagnostics sur l'existence des troubles du rythme, sur la programmation de l'appareil, sur l'efficacité ou le fonctionnement des algorithmes de l'appareil, etc.

Le texte de ces messages est affiché dans des champs d'affichage respectifs distincts 41, 42, 43, 44, ...., éventuellement avec de brèves indications complémentaires, par exemple le paramètre concerné par le message, la valeur actuelle de ce paramètre et la valeur proposée pour une reprogrammation de ce même paramètre afin de remédier à l'anomalie indiquée par le message (l'élaboration de ces différentes indications d'aide au diagnostic est en elle-même connue et ne fait pas partie de l'invention).

Chacun des champs 41, 42, ... comporte un moyen de sélection individuel par le praticien, au choix de ce dernier, par exemple au moyen d'une case respective 51, 52, 53, 54, que le praticien pourra venir pointer ou cocher en y plaçant le curseur de sa souris, selon une technique de commande graphique bien connue. Il est également possible de prévoir une sélection par écran tactile, par un crayon lumineux, par reconnaissance vocale, ou tout autre mode de sélection connu approprié.

On notera par ailleurs que la sélection d'un champ n'est pas nécessairement opérée en cliquant ou en pointant sur une case particulière de ce champ, mais également en cliquant ou en pointant une zone quelconque du champ.

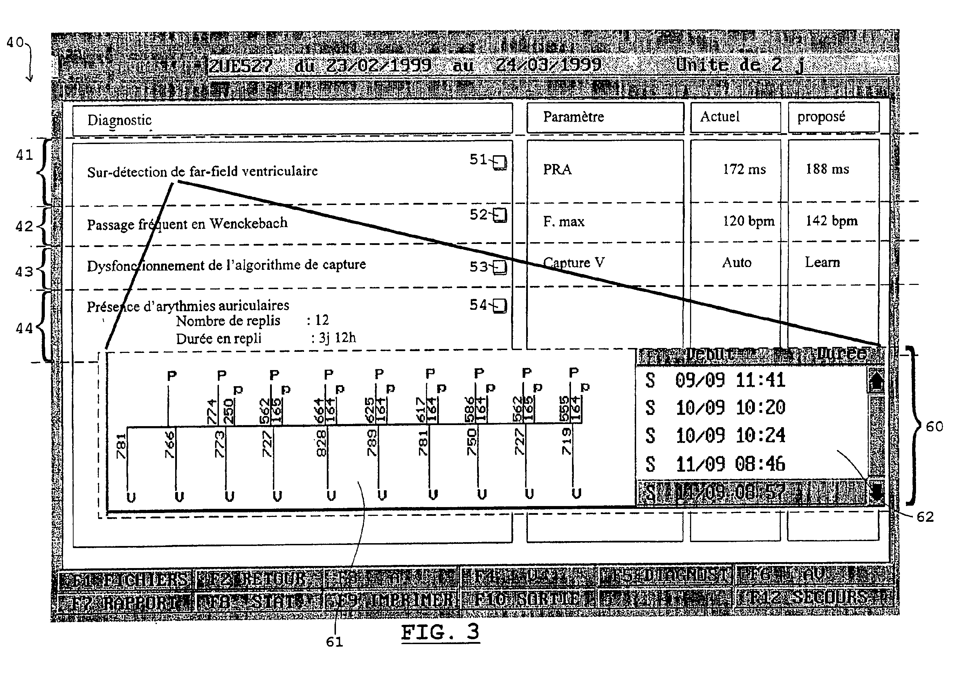

La figure 3 illustre la transformation de l'affichage lorsque le praticien vient, par exemple, sélectionner le champ d'affichage 41 en pointant sur la case associée 51. Cette manoeuvre a pour effet de faire apparaître sur l'écran un bloc 60 comportant un certain nombre de données spécifiques.

Le bloc 60, dont les dimensions sont plus faibles que celles de la page écran 40, est affiché en superposition sur celle-ci ; il peut éventuellement masquer des informations non pertinentes, c'est-à-dire des informations autres que celles du champ d'affichage 41, dans l'exemple décrit.

La sélection par le praticien de l'un des champs d'affichage, c'est-à-dire de l'un des messages d'aide au diagnostic, aura donc pour effet de présenter sur un même écran à la fois le champ sélectionné, qui reste visible, et un certain nombre de données d'implant spécifiques groupées dans un cadre de taille réduite.

Les données spécifiques présentées dans le bloc 60 sont les données d'implant qui ont concouru à l'élaboration de la rubrique du champ d'affichage correspondant.

Dans l'exemple illustré, il s'agit par exemple des données d'implant dont l'analyse par le logiciel a produit un message *"Sur-détection de far-field ventriculaire".* Le praticien peut ainsi, simplement en sélectionnant le champ correspondant à ce message *"Sur-détection de far-field ventriculaire",* faire apparaître les données d'implant pertinentes - et elles seules -- et ainsi affiner son diagnostic par une étude plus fine de ces données. De plus, la visualisation des données spécifiques peut permettre au médecin de vérifier le bien-fondé des diagnostics émis par le logiciel.

On notera que le bloc de données spécifiques est affiché sans commutation de page écran, à la différence de la technique antérieure où, comme illustré figure 1, il était nécessaire au praticien de naviguer dans une arborescence d'écrans pour retrouver les données pertinentes, qui au surplus pouvaient être dispersées sur plusieurs écrans.

Par ailleurs, dans la mesure où les messages affichés dans les différents champs 41, 42, ... correspondent à des symptômes qui sont souvent liés entre eux, il peut être intéressant pour le praticien de passer rapidement d'un bloc de données spécifiques à l'autre, ce qui peut se faire très rapidement et aisément simplement en pointant sur la case 51, 52, ... correspondante, l'écran récapitulatif 40 restant toujours affiché en arrière-plan (ici encore, à la différence de l'art antérieur qui procédait par substitution d'écrans et non par superposition sélective d'informations pertinentes).

Les données spécifiques affichées dans le bloc 60 peuvent se présenter sous différentes formes. Sur la figure 3, on a ainsi illustré une chaîne de marqueurs 61 présentée sous forme d'un chronogramme, et une série horodatée d'événements présentée sous forme d'une liste déroulante 62.

D'autres types de données spécifiques peuvent bien entendu être affichées, sous diverses formes : courbes, histogrammes, etc.

Par ailleurs, la nature et la présentation des données spécifiques varient selon le champ d'affichage sélectionné, puisque seules sont affichées dans le bloc 60 les données spécifiques ayant concouru à l'élaboration de la rubrique correspondante.

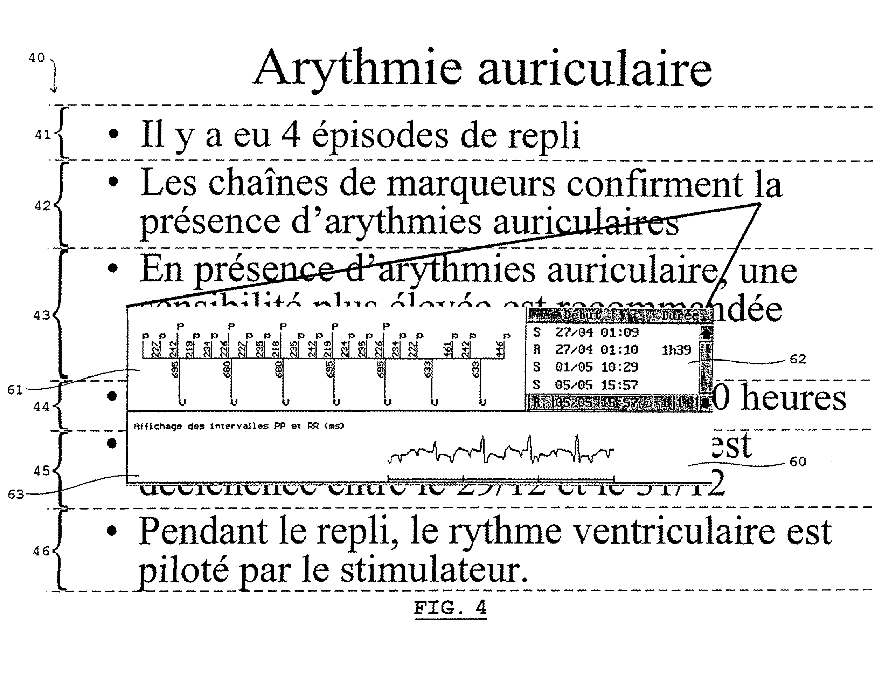

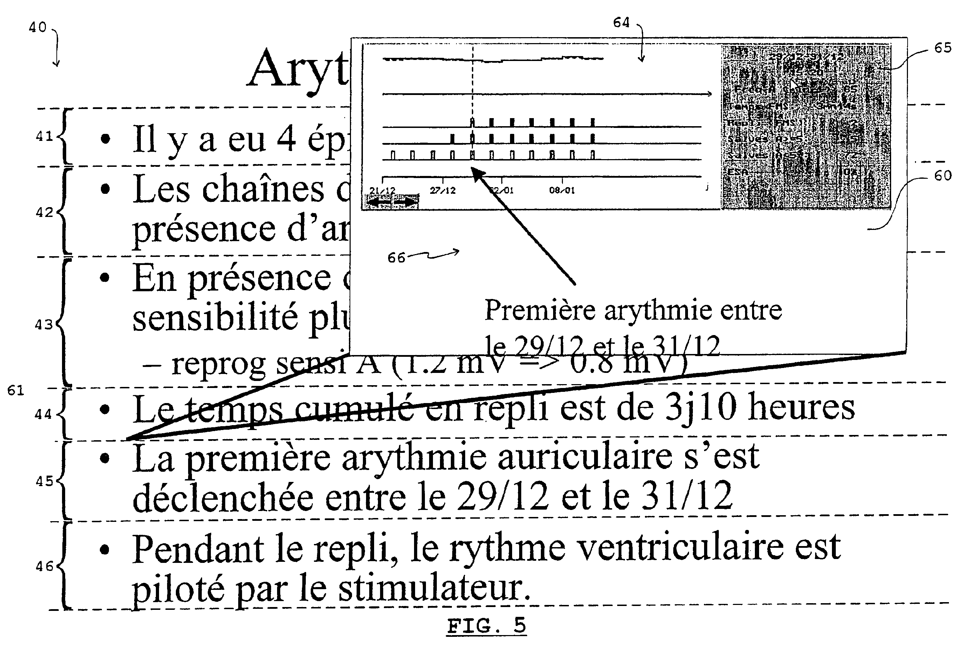

Les figures 4 et 5 illustrent un autre exemple de mise en oeuvre du procédé de l'invention.

Dans cet autre exemple, l'écran récapitulatif 40 se présente simplement sous forme d'une série de messages littéraux. Par exemple, dans le cas d'une arythmie auriculaire, les messages : "*Il y a eu 4 épisodes de repli", "Les chaînes de marqueurs confirment la présence d'arythmies auriculaires*", etc.

Chacun de ces messages correspond à un champ 41, 42, ... 46 sur l'écran.

Le praticien peut sélectionner l'un de ces champs par exemple en plaçant le curseur en un endroit particulier, ou bien quelconque, du champ, ou bien au moyen d'une touche de déplacement de son clavier, ou par commande vocale, par le biais d'écran tactile, etc.

La sélection d'un champ déclenche l'apparition d'un bloc de données spécifiques 60, en superposition sur d'autres champs de l'écran pour laisser apparent le message du champ sélectionné.

Ainsi, sur la figure 4, la sélection du champ 42 fait apparaître une fenêtre 60 masquant partiellement les champs 43, 44 et 45, tandis que sur la figure 5 la sélection du champ 45 fait apparaître une fenêtre 60 masquant les champs 41, 42 et 43.

Dans le cas de la figure 4, la sélection du champ 42 correspondant au message d'aide au diagnostic *"Les chaînes de marqueurs confirment la présence d'arythmies auriculaires"* produit l'affichage d'un bloc de données spécifiques 60 comprenant le chronogramme 61 de la chaîne de marqueurs, la liste horodatée 62 des arythmies qui ont été détectées, avec leur instant d'apparition et leur durée, ainsi qu'un graphique 63 montrant l'évolution des intervalles cardiaques.

Sur la figure 5, la sélection du champ 45 correspondant au message d'aide au diagnostic *"La première arythmie auriculaire s'est déclenchée entre le 29*/*12 et le 31*/*12*" produit l'affichage d'un bloc 60 comprenant une série de chronogrammes 64 reflétant l'évolution du rythme auriculaire, un tableau 65 présentant des résultats statistiques sur la période étudiée (par exemple, comme illustré la fréquence auriculaire, la durée de la période, les épisodes de repli, la répartition des salves et le taux d'extrasystoles), ainsi qu'une flèche 66 pointant sur le chronogramme à l'instant de la survenue de la première arythmie évoquée dans le message du champ 45 (message qui peut d'ailleurs être repris à l'intérieur du bloc 60, comme illustré).

Il est possible que la superposition masque le champ sélectionné. De préférence, la fenêtre 60 peut être cachée ou fermée par un clic de souris ou par une touche (par exemple la touche "Échap") afin de visualiser la totalité de la page écran 40, de manière à faciliter la sélection d'un autre champ sans changer l'écran 40. Les techniques de présentation et de sélection de champs de données sont bien connues des spécialistes de la technique, en utilisant des systèmes d'exploitations classiques à gestion des fenêtres.

## Revendications

1. Un procédé de traitement de données d'implant brutes stockées dans la mémoire d'un dispositif médical implantable actif, notamment d'un stimulateur cardiaque, défibrillateur, cardioverteur et/ou dispositif multisite, procédé **caractérisé par** les étapes consistant à :
a) lire les données d'implant brutes dans la mémoire du dispositif,
b) élaborer des données de synthèse à partir de ces données brutes,
c) répertorier les données de synthèse ainsi élaborées selon une pluralité de rubriques avec, associées à chaque rubrique, un texte de message d'aide au diagnostic et une série de données d'implant spécifiques ayant concouru à l'élaboration de cette rubrique,
d) afficher sur un écran lesdites rubriques, sous forme d'une pluralité de champs d'affichage distincts (41, 42, 43, 44) regroupés sur une même page récapitulative (40), chaque champ comprenant ledit texte de message d'aide au diagnostic correspondant,
e) mettre à disposition de l'utilisateur un moyen de sélection individuelle de chacun desdits champs,
f) lorsqu'un champ de la page récapitulative est sélectionné au choix de l'utilisateur, afficher sur la page récapitulative (40) à la fois le champ sélectionné (41), qui reste visible, et lesdites données d'implant spécifiques (60) ayant concouru à l'élaboration de la rubrique correspondant à ce champ sélectionné (41).

2. Le procédé de la revendication 1, dans lequel les données d'implant spécifiques comprennent des chaînes de marqueurs (61), des histogrammes de fréquences ou d'amplitudes, des courbes et/ou des listes chronologiques horodatées (62) d'événements.

3. Le procédé de la revendication 1, dans lequel l'affichage des données d'implant spécifiques de l'étape f) est une étape d'affichage en superposition sur les champs des rubriques non sélectionnées, la page récapitulative (40) restant affichée en arrière-plan.

4. Le procédé de la revendication 1, dans lequel la sélection de l'étape e) est une sélection par une zone à pointer ou à cocher (51, 52, 53, 54) située à l'intérieur du champ d'affichage (41, 42, 43, 44).

5. Un programmateur, apte à coopérer avec un dispositif médical implantable actif conservant dans sa mémoire des données d'implant brutes, ce programmateur comportant :
- un afficheur graphique,
- un dispositif de pointage,
- un système de télémétrie pour lire lesdites données d'implant brutes, et
- des moyens pour élaborer, à partir de ces données brutes, des données de synthèse répertoriées selon une pluralité de rubriques avec, associées à chaque rubrique, un texte de message d'aide au diagnostic et une série de données d'implant spécifiques ayant concouru à l'élaboration de cette rubrique,
programmateur **caractérisé en ce qu'**il comprend en outre :
- une interface graphique apte à afficher une page écran comprenant lesdites rubriques sous forme d'une pluralité de champs d'affichage distincts (41, 42, 43, 44) regroupés sur une même page récapitulative (40), chaque champ comprenant ledit texte de message d'aide au diagnostic correspondant, chacun de ces champs comportant en outre une zone active et chacune de ces zones actives étant apte à répondre à une sélection de la zone active par le dispositif de pointage en présentant à la fois le champ sélectionné (41), qui reste visible, et les données d'implant spécifiques correspondant au champ choisi sur la page écran, chaque donnée d'implant spécifique étant présentée en superposition sur ladite pluralité de champs.

6. Le programmateur de la revendication 5, dans lequel chaque donnée d'implant spécifique est affichée en superposition sur les champs des rubriques non sélectionnées, la page récapitulative (40) restant affichée en arrière-plan.

7. Le programmateur de la revendication 5, dans lequel chaque donnée d'implant spécifique comprend des données choisies parmi des données littérales, une liste chronologique horodatée d'événements, un histogramme des fréquences cardiaques, un histogramme des amplitudes de stimulation ou de dépolarisation, une chaîne de marqueurs et un électrogramme.

## Claims

1. A method for processing raw implant data stored in the memory of an active implantable medical device, in particular of a cardiac pacemaker, defibrillator, cardiovertor and/or multisite device, said method being **characterised by** the following steps:
a) reading the raw implant data from the memory of the device;
b) elaborating synthesis data from said raw data;
c) indexing the elaborated synthesis data according to a plurality of headings with, associated to each heading, a diagnosis assistance message text and a set of specific implant data having contributed to the elaboration of said heading;
d) displaying said headings on a screen, in the form of a plurality of distinct display fields (41, 42, 43, 44) grouped on a same summary page (40), each field comprising a corresponding one of said diagnosis assistance message text;
e) providing the user with a means for individually selecting each of said fields; and
f) when a field of the summary page is selected by a choice of the user, displaying on the summary page (40) both the selected field (41), which remains visible, and said specific implant data having contributed to the elaboration of the selected heading (41).

2. The method of claim 1, wherein the specific implant data comprises marker chains (61), frequency or amplitude histograms, curves and/or chronological time-stamped lists (62) of events.

3. The method of claim 1, wherein the displaying of the specific implant data of step f) is a step of displaying in superposition over the fields of the non-selected headings, the summary page (40) remaining displayed in the background.

4. The method of claim 1, wherein the selection of step e) is a selection by way of an area to be pointed or ticked (51, 52, 53, 54) located inside the display field (41, 42, 43, 44).

5. A programmer, adapted to interact with an active implantable medial device having a memory with raw implant data stored in it, said programmer including:
- a graphic display;
- a pointing device;
- a telemetry system for reading said raw implant data; and
- means for elaborating, from said raw data, synthesis data indexed according to a plurality of headings with, associated to each heading, a diagnosis assistance message text and a set of specific implant data having contributed to the elaboration of said heading,
said programmer being **characterised by** further comprising:
g) a graphic user interface adapted to display a screen-page comprising said headings in the form of a plurality of distinct display fields (41, 42, 43, 44) grouped on a same summary page (40), each field comprising a corresponding one of said diagnosis assistance message text, each said field further comprising an active area and each of said active area being adapted to respond to a selection of the active zone through said pointing device by presenting both the selected field (41), which remains visible, and the specific implant data corresponding to the field selected on the screen-page, each set of specific implant data being presented superimposed over said plurality of fields.

6. The programmer of claim 5 wherein each set of specific implant data is displayed in superposition over the fields of the non-selected headings, the summary page (40) remaining displayed in the background.

7. The programmer of claim 5 wherein each set of specific implant data comprises data selected among text data, a chronologic time-stamped list of events, a cardiac frequency histogram, a cardiac depolarisation or stimulation amplitude histogram, a marker chain, and an electrogram.

## Patentansprüche

1. Ein Verfahren zur Bearbeitung von Rohdaten eines Implantats, welche in dem Speicher einer aktiven implantierbaren medizinischen Vorrichtung insbesondere eines Herzschrittmachers, Defibrillators, eines Kardioverters und/oder Multisitvorrichtung gespeichert ist, wobei das Verfahren durch die Arbeitsschritte **gekennzeichnet** ist, welche bestehen aus:
a) dem Lesen der Rohdaten des Implantats in dem Speicher der Vorrichtung,
b) dem Erstellen von zusammengefassten Daten, ausgehend von diesen Rohdaten,
c) dem Verzeichnen der so erstellten zusammengefassten Daten gemäß einer Vielzahl von Rubriken mit, jeder Rubrik zugeordnet, einem Hilfsmitteilungstext zur Diagnose und einer Reihe von spezifischen Daten des Implantats, welche zum Erstellen dieser Rubriken beitragen,
d) dem Anzeigen der Rubriken auf einem Bildschirm in Gestalt einer Vielzahl von verschiedenen Anzeigefeldern (41, 42, 43, 44), welche auf einer selben zusammenfassenden Seite (40) angeordnet sind, wobei jedes Feld den Hilfsmitteilungstext zur entsprechenden Diagnose mit einschließt,
e) dem Bereitstellen eines individuellen Auswahlmittels für jedes der Felder für den Benutzer,
f) wenn ein Feld der zusammenfassenden Seite auf Wahl des Benutzers ausgewählt ist, dem Anzeigen zugleich des ausgewählten Feldes (41), das sichtbar bleibt, und der spezifischen Daten des Implantats (60) auf der zusammenfassenden Seite (40), welche zum Erstellen der Rubrik, welche dem gewählten Feld (41) entspricht, beitragen.

2. Das Verfahren des Anspruchs 1, in welchem die spezifischen Daten des Implantats Markerketten (61), Frequenz- oder Amplitudenhistogramme, Kurven und/oder chronologische datierte Listen (62) der Ereignisse mit einschließen.

3. Das Verfahren des Anspruchs 1, in welchem die Anzeige der spezifischen Daten des Implantats des Schritts f) ein Anzeigeschritt in Überlagerung auf die nicht ausgewählten Rubrikfelder ist, wobei die zusammenfassende Seite (40) im Hintergrund angezeigt bleibt.

4. Das Verfahren von Anspruch 1, in welchem die Auswahl des Schritts e) eine Auswahl durch Anklickfeld oder durch ein Ankreuzfeld (51, 52, 53, 54), welches sich im Inneren des Anzeigefelds (41, 42, 43, 44) befindet, ist.

5. Eine Programmiervorrichtung, welche geeignet ist, mit einer aktiven programmierbaren medizinischen Vorrichtung, welche in ihrem Speicher Rohdaten des Implantats aufbewahrt, zusammenzuwirken, wobei die Programmiervorrichtung einschließt:
- eine graphische Anzeigevorrichtung,
- eine Anklickvorrichtung,
- eine Telemetriesystem, um die Rohdaten des Implantats zu lesen und Mittel, um, ausgehend von diesen Rohdaten, verzeichnete zusammenfassende Daten gemäß einer Vielzahl von Rubriken mit, zu jeder Rubrik zugeordnet, einem Hilfsmitteilungstext zur Diagnose und einer Reihe von spezifischen Daten des Implantats, welche zum Erstellen dieser Rubrik beitragen, zu erstellen, wobei die Programmiervorrichtung **dadurch gekennzeichnet ist, dass** sie unter anderem mit einschließt:
- eine graphische Schnittstelle, dazu geeignet, eine Bildschirmseite anzuzeigen, welche die Rubriken in der Form einer Vielzahl von einzelnen Anzeigefeldern (41, 42, 43, 44) einschließt, welche auf derselben zusammenfassenden Seite (40) angeordnet sind, wobei jedes Feld die entsprechenden Hilfstextmitteilungen zur Diagnose mit einschließt, wobei jedes dieser Felder unter anderem einen aktiven Bereich mit einschließt und jeder dieser aktiven Bereiche geeignet ist, um auf eine Auswahl des aktiven Bereichs durch die Anklickvorrichtung zu antworten, indem zugleich das ausgewählte Feld (41), welches sichtbar bleibt und die spezifischen Daten des Implantats, welche dem auf der Bildschirmfläche ausgewählten Feld entsprechen, anzuzeigen, wobei alle spezifischen Daten des Implantats in Überlagerung mit der Vielzahl der Felder dargestellt wird.

6. Die Programmiervorrichtung aus Anspruch 5, in welcher alle spezifischen Daten des Implantats überlagert zu den Feldern der nicht ausgewählten Rubriken dargestellt werden, wobei die zusammenfassende Seite (40) im Hintergrund angezeigt bleibt.

7. Die Programmiervorrichtung aus Anspruch 5, in welcher alle spezifischen Daten des Implantats Daten, die unter wortgetreuen Daten ausgewählt wurden, eine datierte chronologische Liste der Ereignisse, ein Histogramm der Herzfrequenzen und ein Histogramm der Stimulations- und Depolarisationsamplituden, eine Markerkette und ein Elektrogramm mit einschließen.
